# EUROPEAN PATENT APPLICATION

(11) **EP 4 600 361 A1**
(43) Date of publication of application: **13.08.2025**
(21) Application number: 23874976.6
(22) Date of filing: 06.10.2023
(51) Int. Cl.: C12N 15/62, A61K 39/00, A61P 37/04, C07K 14/08, C07K 14/115, C07K 14/165, C07K 14/54, C07K 19/00, C12N 15/12, C12N 15/13, C12N 15/24, C12N 15/40, C12N 15/45, C12N 15/50

(54) **DESIGN METHOD AND PRODUCTION METHOD FOR NUCLEIC ACID CONSTRUCT AND PROTEIN COMPLEX**

(30) Priority: 07.10.2022 JP 2022162824
(71) Applicant: University of Tsukuba, Ibaraki 305-8577 (JP)
(72) Inventor: OKAMOTO, Tadao, Tsukuba-shi, Ibaraki 305-8577 (JP); TAKADA, Hidetoshi, Tsukuba-shi, Ibaraki 305-8577 (JP)
(74) Representative: ABG Intellectual Property Law, S.L.
(86) International application number: PCT/JP2023/036654
(87) International publication number: WO 2024/075851

(57) **Abstract**

An object of the present invention is to provide a novel nucleic acid construct that induces antibody production. According to the present invention, there is provided a nucleic acid construct that induces production of an antibody against a target protein, the nucleic acid construct comprising: a polynucleotide encoding two or more T cell epitopes; and a polynucleotide encoding one or more B cell epitopes of the target protein. According to the present invention, it is possible to strongly induce the antibody against the target protein in the body of a subject, and thus the present invention is advantageous in that it is possible not only to induce production of an antibody against a foreign antigen such as a bacterium or a virus, but also to induce production of an antibody against a protein produced in a subject's body.

## Description

### CROSS-REFERENCE TO RELATED APPLICATION

The present application enjoys the benefit of priority from the prior Japanese Patent Application No. 2022-162824 filed on October 7, 2022, the entire disclosure of which is incorporated herein by reference.

### TECHNICAL FIELD

The present invention relates to a nucleic acid construct, and a design method and a production method for the same.

### BACKGROUND ART

Many antibody drugs exhibit excellent therapeutic effects in the treatment of chronic diseases and intractable diseases, but many of their target molecules are self-proteins (e.g., inflammatory cytokines, which are causative agents of autoimmune diseases). On the other hand, vaccines induce effective antibodies against foreign antigens such as viruses, and are used primarily to treat infectious diseases. However, existing vaccine technologies, disadvantageously, cannot be applied to the treatment of chronic diseases or intractable diseases because they cannot induce production of antibodies against self-proteins due to immune tolerance (Non-Patent Documents 1, 2, 3 and 4).

### Reference List

### Non-Patent Documents

Non-Patent Document 1: C A Janeway Jr., Immunol Today; 13(1): 11-6 (1992).
Non-Patent Document 2: Ada G., N Engl J Med; 345(14): 1042-53 (2001).
Non-Patent Document 3: Ada GL., Lancet; 335(8688): 523-6 (1990).
Non-Patent Document 4: Anderson RM, et al., Lancet; 350(9089): 1466-70 (1997).

### SUMMARY OF THE INVENTION

### Technical Problem

An object of the present invention is to provide a novel nucleic acid construct that induces antibody production, and methods for designing and producing the same. Solution to Problem

The present inventors have discovered that antibody production can be induced by a nucleic acid construct comprising: a polynucleotide encoding two or more T cell epitopes; and a polynucleotide encoding one or more B cell epitopes of the target protein. The present inventors have also found that the nucleic acid construct is effective as a vaccine through a test of administration of the nucleic acid construct to animals. The present invention is based on the findings.

According to the present invention, the following inventions are provided.
[1] A nucleic acid construct that induces production of an antibody against a target protein, the nucleic acid construct comprising: a polynucleotide encoding two or more T cell epitopes; and a polynucleotide encoding one or more B cell epitopes of the target protein.
[2] The nucleic acid construct according to [1], wherein the two or more T cell epitopes each consist of an amino acid sequence selected from the group consisting of the amino acid sequences set forth in SEQ ID NOs: 1 to 82, or an amino acid sequence substantially identical with the amino acid sequence.
[3] The nucleic acid construct according to [1] or [2], wherein the target protein is a protein produced in a subject's body.
[4] The nucleic acid construct according to any one of [1] to [3], wherein the target protein is IL-17A and/or IL-23.
[5] The nucleic acid construct according to any one of [1] to [4], wherein the target protein is a protein that causes a disease or a protein that is produced due to a disease, and wherein the disease is an autoimmune disease or allergic disease.
[6] The nucleic acid construct according to [5], wherein the autoimmune disease is one or more autoimmune diseases selected from the group consisting of psoriasis, rheumatoid arthritis, systemic lupus erythematosus, Graves' disease, chronic thyroiditis, type I diabetes, vasculitis, Addison's disease, polymyositis, Sjogren's syndrome, systemic sclerosis and glomerulonephritis.
[7] The nucleic acid construct according to [5], wherein the allergic disease is one or more allergic diseases selected from the group consisting of atopic dermatitis, bronchial asthma, allergic rhinitis, hay fever, food allergy and allergic conjunctivitis.
[8] The nucleic acid construct according to any one of [1] to [7], which is DNA or RNA.
[9] An agent for preventing or treating a disease, comprising the nucleic acid construct according to any one of [1] to [8] as an active ingredient.
[10] The agent according to [9], wherein the disease is an autoimmune disease or allergic disease.
[11] The agent for preventing a disease according to [9] or [10], which is a pan-HLA compatible vaccine.
[12] A method for producing an agent for preventing or treating a disease, the method comprising the step of using a nucleic acid construct that induces production of an antibody against a target protein as an active ingredient, wherein the nucleic acid construct comprises: a polynucleotide encoding two or more T cell epitopes; and a polynucleotide encoding one or more B cell epitopes of the target protein.
[13] The production method according to [12], wherein the disease is an autoimmune disease or allergic disease.
[14] A method for designing a nucleic acid construct or protein complex that induces production of an antibody against a target protein, the method comprising the step of combining two or more T cell epitopes and one or more B cell epitopes of the target protein.
[15] A method for producing an agent for preventing or treating a disease, the method comprising the following steps:
   (P) the step of carrying out the design method according to claim 14 to design a nucleic acid construct or protein complex that induces production of an antibody against a target protein; and
   (Q) the step of preparing the nucleic acid construct or protein complex designed in the step (P).
[101] A method for designing a nucleic acid construct or protein complex that induces production of an antibody against a target protein, the method comprising the step of combining two or more T cell epitopes and one or more B cell epitopes of the target protein.
[102] The design method according to [101], wherein the T cell epitopes have a high ability to bind to an MHC class II molecule.
[103] The design method according to [101] or [102], wherein the target protein is a protein that causes a disease or a protein that is produced due to a disease.
[104] The design method according to any one of [101] to [103], wherein the target protein is a protein produced in a subject's body.
[105] The design method according to [103] or [104], wherein the disease is one or more diseases selected from the group consisting of autoimmune diseases, allergic diseases, cancer, neurodegenerative diseases, infectious diseases and autoinflammatory diseases.
[106] The design method according to [105], wherein the autoimmune disease is one or more autoimmune diseases selected from the group consisting of psoriasis, rheumatoid arthritis, systemic lupus erythematosus, Graves' disease, chronic thyroiditis, type I diabetes, vasculitis, Addison's disease, polymyositis, Sjogren's syndrome, systemic sclerosis and glomerulonephritis.
[107] The design method according to [105], wherein the allergic disease is one or more allergic diseases selected from the group consisting of atopic dermatitis, bronchial asthma, allergic rhinitis, hay fever, food allergy and allergic conjunctivitis.
[108] The design method according to [105], wherein the cancer is one or more types of cancer selected from the group consisting of skin cancer, colon cancer, breast cancer, gastric cancer, leukemia, malignant lymphoma and multiple myeloma.
[109] The design method according to [105], wherein the neurodegenerative disease is Alzheimer's disease.
[110] The design method according to [105], wherein the infectious disease is one or more infectious diseases selected from the group consisting of Zika fever, SARS-CoV-2 infection, and influenza infection.
[111] The design method according to [105], wherein the autoinflammatory disease is one or more autoinflammatory diseases selected from the group consisting of familial Mediterranean fever, cryopyrin-associated periodic syndrome, TNF receptorassociated periodic syndrome, mevalonate kinase deficiency, Blau syndrome, periodic fever, aphthous stomatitis, pharyngitis and cervical adenitis syndrome.
[112] The design method according to [103], wherein the disease is one or more diseases selected from the group consisting of multiple sclerosis, neuromyelitis optica, myasthenia gravis, pemphigus and autoimmune hemolytic anemia.
[113] The design method according to any one of [101] to [112], wherein the target protein is one or more proteins selected from the group consisting of IL-17A, IL-23, IL-1β, IL-13, IL-13R, IL-4Rα, TNF-α, IL-6, IL-6R, IgE, IL-5, IL-5Rα, FcRn, PD-1, PD-L1, CTLA-4, HER2, EGFR, CD22, CD20, RANKL, C5, IFNγ, amyloid β, respiratory syncytial (RS) virus antigen protein, Zika virus antigen protein, SARS-CoV-2 virus antigen protein and influenza virus antigen protein.
[114] The design method according to any one of [101] to [112], wherein the target protein is IL-17A and/or IL-23.
[115] The design method according to any one of [101] to [114], wherein the nucleic acid construct is DNA or mRNA.
[116] A method for producing an agent for preventing or treating a disease, the method comprising the following steps:
   (P) the step of carrying out the design method according to any one of [101] to [115] to design a nucleic acid construct or protein complex that induces production of an antibody against a target protein; and
   (Q) the step of preparing the nucleic acid construct or protein complex designed in the step (P).

According to the present invention, it is possible to strongly induce the antibody against the target protein in the body of a subject, and thus the present invention is advantageous in that it is possible not only to induce production of an antibody against a foreign antigen such as a bacterium or a virus, but also to induce production of an antibody against a protein produced in a subject's body (self-protein) (for example, an inflammatory cytokine produced in the body of the subject who has developed an autoimmune disease or the like).

### BRIEF DESCRIPTION OF THE DRAWINGS

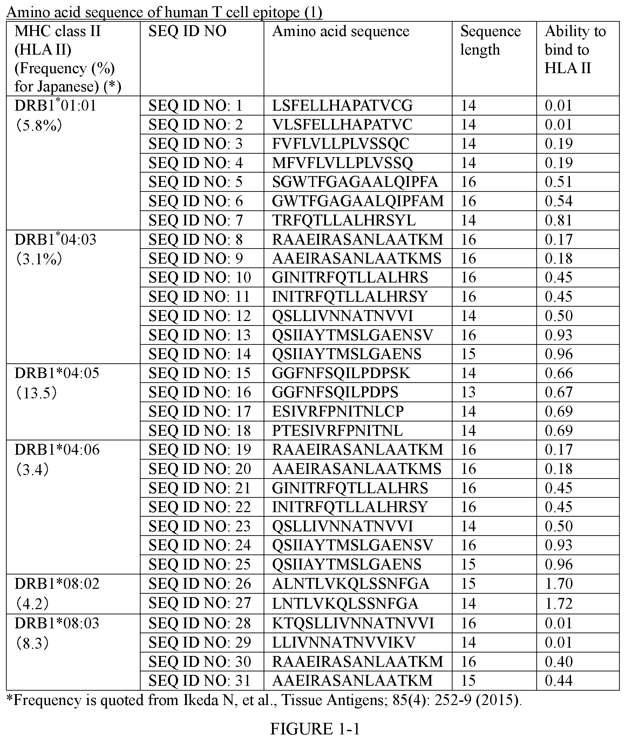

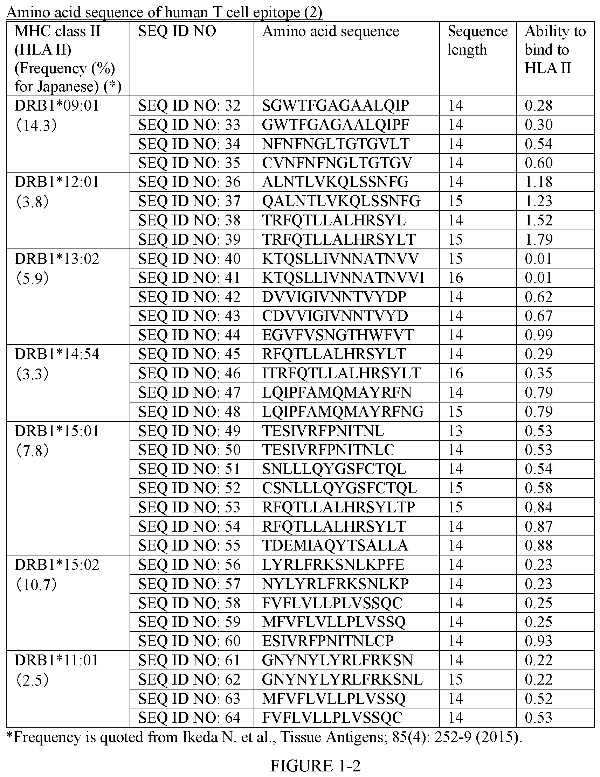

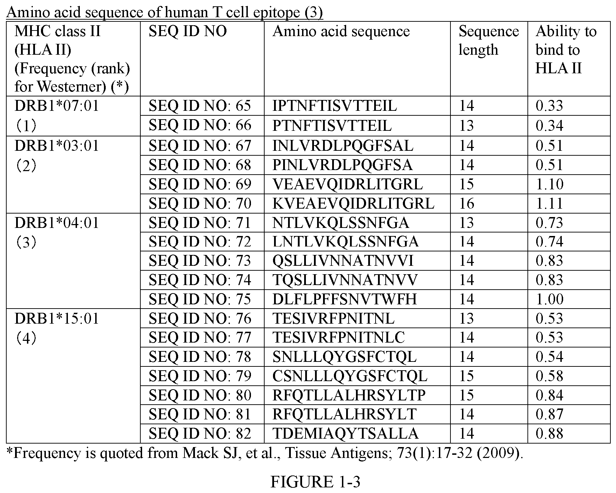

FIG. 1-1 shows specific examples of amino acid sequences of human T cell epitopes.
FIG. 1-2 shows specific examples of amino acid sequences of human T cell epitopes.
FIG. 1-3 shows specific examples of amino acid sequences of human T cell epitopes.
FIG. 2A is an image view of the composition of a nucleic acid construct (mRNA vaccine) of the present invention. FIG. 2B is a schematic diagram of a nucleic acid construct prepared in the Examples (Example 3). T-86 to T88 correspond to base sequences encoding T cell epitopes set forth in SEQ ID NOs: 86 to 88, respectively, and B89 to 94 correspond to base sequences encoding B cell epitopes set forth in SEQ ID NOs: 89 to 94, respectively.
FIG. 3 shows antibody titers (absorbance at a wavelength of 450 nm) of antibodies against IL-17A and IL-23, the production of which was induced by the nucleic acid constructs encoding T cell epitopes. An error bar indicates a standard deviation.
FIG. 4 shows antibody titers (absorbance at a wavelength of 450 nm) of subclass antibodies against IL-17A and IL-23, the production of which was induced by the nucleic acid constructs encoding T cell epitopes. IgG1, IgG2a, IgG2b and IgG3 all indicate antibody titers of an administration group.
FIG. 5 shows antibody titers (absorbance at a wavelength of 450 nm) of antibodies against IL-17A and IL-23, the production of which was induced by the nucleic acid constructs encoding T cell epitopes. An error bar indicates a standard deviation.
FIG. 6A shows changes in ear thickness of a control group after application of IMQ and normal mice. FIG. 6B shows inhibition rates of ear thickness increase 5 days after application of IMQ in the control group and the administration group. FIG. 6C shows representative photographs of the ears of psoriasis model mice (the control group and the administration group) 6 days after application of IMQ and normal mice.
FIG. 7A shows changes in ear thickness of the control group after application of IMQ and normal mice. FIG. 7B shows inhibition rates of ear thickness increase 6 days after application of IMQ in the control group and the administration group. FIG. 7C shows representative photographs of the ears of the psoriasis model mice (the control group and the administration group) 6 days after application of IMQ and normal mice.

### DETAILED DESCRIPTION OF THE INVENTION

### <<Nucleic acid construct>>

According to the present invention, a nucleic acid construct is provided. The nucleic acid construct of the present invention is a nucleic acid construct that induces production of an antibody against a target protein, the nucleic acid construct being characterized by comprising: a polynucleotide encoding two or more T cell epitopes; and a polynucleotide encoding one or more B cell epitopes of the target protein.

In the present invention, the term "T cell epitope" refers to a peptide that binds to a major histocompatibility complex (MHC) class II molecule, among MHCs, to form a complex which is displayed on antigen-presenting cells (e.g., B cells, dendritic cells, and macrophages) and specifically recognized by a T cell receptor (TCR) of CD4-positive T cells.

In the present invention, any types of T cell epitopes can be selected as the two or more T cell epitopes, as long as they bind to the target MHC class II molecule. However, from the viewpoint of strongly inducing production of an antibody against a target protein (i.e., strongly activating CD4-positive T cells), it is preferable to select T cell epitopes that are taken up by antigen-presenting cells in the living body of a subject and strongly bind to an MHC class II molecule (i.e., have a high ability to bind to an MHC class II molecule).

In the present invention, the T cell epitopes having a high ability to bind to an MHC class II molecule can be selected using a known MHC binding prediction tool with a predicted binding score for MHC class II as an index. Examples of the MHC binding prediction tool include immune epitope database analysis resource (IEDB Analysis Resource, http://tools.iedb.org/main/), MHCBN (http://crdd.osdd.net/raghava/mhcbn/), NetMHCII (https://services.healthtech.dtu.dk/service.php?NetMHCII-2.3), SYFPEITHI (http://www.syfpeithi.de/0-Home.htm), ANTIJEN (http://www.ddg-pharmfac.net/antijen/AntiJen/antijenhomepage.htm), IMGT/3Dstructure-DB (https://www.imgt.org/IMGTindex/IMGT3Dstructure-db.php), and SEDB (http://sedb.bicpu.edu.in/).

In the case of humans, MHC, as used herein, refers to a human leukocyte antigen (HLA), and examples of the HLA class II molecule include HLA-DR, HLA-DQ and HLA-DP. HLA-DR, HLA-DQ and HLA-DP are all composed of an α chain and a β chain. Examples of the α chain include HLA-DRA, HLA-DQA and HLA-DPA, and examples of the β chain include HLA-DRB, HLA-DQB and HLA-DPB.

Examples of HLA-DR include HLA-DR1, HLA-DR2, HLA-DR3, HLA-DR4, HLA-DR5, HLA-DR6, HLA-DR7, HLA-DR8, HLA-DR9, HLA-DR10, HLA-DR11, HLA-DR12, HLA-DR13, HLA-DR14, HLA-DR15, HLA-DR52, and HLA-DR53.

Examples of HLA-DQ include HLA-DQ1, HLA-DQ2, HLA-DQ3, HLA-DQ4, HLA-DQ5, HLA-DQ6, HLA-DQ7, and HLA-DQ8.

Examples of HLA-DP include HLA-DP1, HLA-DP2, HLA-DP3, HLA-DP4, and HLA-DP5.

In the present invention, HLA-DR, as an example of the HLA to which the T cell epitopes bind, is composed of an allele such as HLA-DRA1*01 as an α chain and an allele such as DRB1*01:01, DRB1*04:03, DRB1*04:05, DRB1*04:06, DRB1*08:02, DRB1*08:03, DRB1*0 9:01, DRB1*12:01, DRB1*13:02, DRB1*14:54, DRB1*15:01, DRB1*15:02 or DRB1*11:01 as a β chain in the case of a Japanese subject, and composed of an allele such as HLA-DRA1*01 as an α chain and DRB1*07:01, DRB1*03:01, DRB1*04:01 or DRB1*15:01 as a β chain in the case of a Western subject.

In the present invention, specific examples of the T cell epitopes include, but are not limited to, those shown in FIG. 1-1 (SEQ ID NOs: 1 to 31), FIG. 1-2 (SEQ ID NOs: 32 to 64), and FIG. 1-3 (SEQ ID NOs: 65 to 82). Namely, in the present invention, the two or more T cell epitopes can each consist of an amino acid sequence selected from the group consisting of the amino acid sequences set forth in SEQ ID NOs: 1 to 82. In the present invention, the T cell epitopes can also each consist of an amino acid sequence selected from the group consisting of the amino acid sequences set forth in SEQ ID NOs: 1 to 64 or an amino acid sequence selected from the group consisting of the amino acid sequences set forth in SEQ ID NOs: 65 to 82.

In the present invention, the T cell epitopes can each consist of an amino acid sequence substantially identical with an amino acid sequence selected from the amino acid sequences set forth in SEQ ID NOs: 1 to 82 (or the amino acid sequences set forth in SEQ ID NOs: 1 to 64 or SEQ ID NOs: 65 to 82).

In the present invention, the "T cell epitope consisting of a substantially identical amino acid sequence," as used herein, refers to a T cell epitope consisting of a specific amino acid sequence, which contains one or more modifications and retains the ability to bind to an MHC class II molecule (HLA class II molecule).

In the present invention, examples of the T cell epitope consisting of a substantially identical amino acid sequence include an amino acid sequence selected from the amino acid sequences set forth in SEQ ID NOs: 1 to 82 (or the amino acid sequences set forth in SEQ ID NOs: 1 to 64 or SEQ ID NOs: 65 to 82), which has one or more modifications selected from the group consisting of deletions, substitutions, insertions, and additions and has an ability to bind to an MHC class II molecule (HLA class II molecule). The number of amino acids to be modified is, for example, 1 to 4 or 1 to 3, particularly preferably 2 or 1. The number of amino acids to be modified can be equivalent to the number of mutations occurring by a known method such as sitespecific mutagenesis, or equivalent to the number of naturally occurring mutations. The modifications may occur consecutively or discontinuously in the amino acid sequence. The modifications may also be a plurality of modifications of the same type (e.g., a plurality of substitutions) or a plurality of modifications of different types (e.g., a combination of one or more deletions and one or more substitutions).

The modification of amino acids may be a conservative modification. The term "conservative modification" means that one or more amino acids are modified in such a manner that the functions of the protein are not substantially modified. The substitution of amino acids may also be a conservative substitution. The term "conservative substitution" means that one or more amino acids are substituted with other amino acids and/or amino acid derivatives in such a manner that the functions of the protein are not substantially modified. In the conservative substitution, the amino acid to be substituted and the substituted amino acid are preferably similar in properties and/or functions, for example. Specifically, these amino acids are preferably similar in chemical properties, such as hydrophobicity and hydrophilicity indices, polarity, and charge, or physical properties, such as secondary structure. Thus, amino acids or amino acid derivatives having similar properties and/or functions are known in the art. Examples of non-polar amino acids (hydrophobic amino acids) include alanine, valine, isoleucine, leucine, proline, tryptophan, phenylalanine, and methionine. Examples of polar amino acids (neutral amino acids) include glycine, serine, threonine, tyrosine, glutamine, asparagine, and cysteine. Examples of positively charged amino acids (basic amino acids) include arginine, histidine, and lysine, and examples of negatively charged amino acids (acidic amino acids) include aspartic acid and glutamic acid.

In the present invention, examples of the T cell epitope consisting of a substantially identical amino acid sequence include those which have 80% or more (preferably 81% or more, 82% or more, 83% or more, 84% or more, 85% or more, 86% or more, 87% or more, 88% or more, 89% or more, more preferably 90% or more, 91% or more, 92% or more, 93% or more, or 94% or more, and even more preferably 95% or more, 96% or more, 97% or more, 98% or more, or 99% or more) sequence identity with an amino acid sequence selected from the amino acid sequences set forth in SEQ ID NOs: 1 to 82 (or the amino acid sequences shown in SEQ ID NOs: 1 to 64 or SEQ ID NOs: 65 to 82) and have an ability to bind to an MHC class II molecule (HLA class II molecule). The term "identity," as used herein, refers to the degree of identity when the sequences to be compared are appropriately aligned, and means the appearance rate (%) of exact amino acid matches between the sequences. For example, the presence of gaps in the sequence and the properties of amino acids are taken into consideration in the calculation of the identity (Wilbur, Natl. Acad. Sci. U.S.A. 80:726-730 (1983)). The alignment can be performed, for example, by using any algorithm. Specifically, publicly available homology search software such as BLAST (Basic local alignment search tool) (Altschul et al., J. Mol. Biol. 215:403-410 (1990)), FASTA (Peasron et al., Methods in Enzymology 183:63-69 (1990)), and Smith-Waterman (Meth. Enzym., 164, 765 (1988)) can be used. Further, the identity can be calculated, for example, using a publicly available homology search program as described above. For example, the identity can be calculated using default parameters in the homology algorithm BLAST (https://blast.ncbi.nlm.nih.gov/Blast.cgi) of the National Center for Biotechnology Information (NCBI).

The T cell epitopes to be combined in the present invention can be selected from, for example, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, and 20 or more T cell epitopes. For example, the more types of T cell epitopes to be combined, the higher the probability that T cell epitopes with a high ability to bind to the target MHC class II molecule will be included in the combination. Therefore, when the nucleic acid construct and protein complex of the present invention are used as active ingredients of a vaccine, the vaccine can be administered to a broader range of subjects, which will therefor contribute to universalization of vaccines. In the present invention, the term "protein complex," as used herein, refers to a fusion protein (typically, a protein that induces production of an antibody against the target protein) comprising T cell epitopes and B cell epitopes as components, the fusion protein being produced by translation of the nucleic acid construct of the present invention.

In the present invention, specific examples of the combination of the amino acid sequences of the T cell epitopes include combinations consisting of 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more amino acid sequences selected from the group consisting of the amino acid sequences set forth in SEQ ID NOs: 1 to 64 or amino acid sequences substantially identical with the amino acid sequences. Furthermore, the above-described combination can be, but is not limited to, a combination of 64 or less, 60 or less, 55 or less, 50 or less, 45 or less, 40 or less, 35 or less, or 30 or less amino acid sequences. These lower and upper limit values can be combined arbitrarily.

In the present invention, specific examples of the combination of the amino acid sequences of the T cell epitopes include combinations consisting of 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more amino acid sequences selected from the group consisting of the amino acid sequences set forth in SEQ ID NOs: 1, 2, 7 to 9, 13 to 20, 24 to 27, 30, 31, 34 to 37, 42 to 44, 47 to 54, 56, 57 and 60 to 62 or amino acids substantially identical with the amino acid sequences. Furthermore, the above-described combination can be, but is not limited to, a combination of 39 or less, 35 or less, 30 or less, or 25 or less amino acid sequences. These lower and upper limit values can be combined arbitrarily.

In the present invention, the T cell epitopes can also be a combination of amino acid sequences of T cell epitopes that bind to different MHC class II molecules (HLA class II molecules). Specific examples of such a combination include combinations of two or more amino acid sequences selected from at least one, two, three, four or five selected from the group consisting of the amino acid sequences set forth in SEQ ID NOs: 1 to 7, at least one, two, three, four or five selected from the group consisting of the amino acid sequences set forth in SEQ ID NOs: 8 to 14, at least one, two or three selected from the group consisting of the amino acid sequences set forth in SEQ ID NOs: 15 to 18, at least one, two, three, four or five selected from the group consisting of the amino acid sequences set forth in SEQ ID NOs: 19 to 25, at least one selected from the group consisting of the amino acid sequences set forth in SEQ ID NOs: 26 and 27, at least one, two or three selected from the group consisting of the amino acid sequences set forth in SEQ ID NOs: 28 to 31, at least one, two or three selected from the group consisting of the amino acid sequences set forth in SEQ ID NOs: 32 to 35, at least one, two or three selected from the group consisting of the amino acid sequences set forth in SEQ ID NOs: 36 to 39, at least one, two or three selected from the group consisting of the amino acid sequences set forth in SEQ ID NOs: 40 to 44, at least one, two or three selected from the group consisting of the amino acid sequences set forth in SEQ ID NOs: 45 to 48, at least one, two, three, four or five selected from the group consisting of the amino acid sequences set forth in SEQ ID NOs: 49 to 55, at least one, two or three selected from the group consisting of the amino acid sequences set forth in SEQ ID NOs: 56 to 60, and at least one or two selected from the group consisting of the amino acid sequences set forth in SEQ ID NOs: 61 to 64.

In the present invention, the T cell epitopes can also be a combination of amino acid sequences of T cell epitopes that bind to different MHC class II molecules (HLA class II molecules). Specific examples of such a combination include combinations of two or more amino acid sequences selected from the group consisting of at least one selected from the group consisting of the amino acid sequences set forth in SEQ ID NOs: 65 and 66, at least one, two or three selected from the group consisting of the amino acid sequences set forth in SEQ ID NOs: 67 to 70, at least one, two or three selected from the group consisting of the amino acid sequences set forth in SEQ ID NOs: 71 to 75, and at least one, two, three, four or five selected from the group consisting of the amino acid sequences set forth in SEQ ID NO: 76 to 82.

As described above, since it is possible to combine amino acid sequences of T cell epitopes that bind to different MHC class II molecules (HLA class II molecules), the nucleic acid construct or protein complex of the present invention can be used as an active ingredient of a pan-HLA compatible vaccine. In the present invention, the term "pan-HLA compatible vaccine," as used herein, refers to a vaccine that can be widely applied to the subject, and specifically refers to a vaccine configured to recognize two or more different HLA class II molecules present in humans.

The nucleic acid construct or protein complex of the present invention may contain a plurality of sets of T cell epitopes of the same type, from the viewpoint of enhancing induction of an antibody against the target protein in the subject. As presented in the Examples (Example 3) below, the nucleic acid construct may be configured to contain base sequences encoding T cell epitopes of the same type a plurality of times repeatedly, or may be configured to contain the base sequences a plurality of times randomly (for example, in the Examples (Example 3) below, the nucleic acid construct is configured to contain each of the base sequences of SEQ ID NOs: 86 to 88 three times repeatedly).

In the present invention, the term "B cell epitope" means a moiety (peptide) of a target protein (antigen) that is recognized by an antigen-specific B cell.

In the present invention, any types of B cell epitopes can be selected as the B cell epitopes to be combined, as long as they are recognized by antigen-specific B cells.

In the present invention, the target protein includes, but is not limited to, proteins that cause a disease and proteins that are produced due to a disease, including both foreign antigens (bacteria, viruses, and the like) and proteins produced in a subject's body (self-proteins) (such as inflammatory cytokines produced in the body of the subject who has developed an autoimmune disease or the like). In the present invention, the term "protein that causes a disease," as used herein, refers to a protein that is a source of causing a disease, and includes proteins that are sources of suffering from a disease or developing a disease. Furthermore, the term "protein that is produced due to a disease" refers to a protein produced due to the development of a disease as a trigger, and, in particular, to a protein that has an action of exacerbating or aggravating symptoms of the disease (e.g., an inflammatory protein).

In the present invention, examples of diseases associated with the target protein include autoimmune diseases, allergic diseases, cancer, neurodegenerative diseases, infectious diseases, and autoinflammatory diseases.

In the present invention, examples of the autoimmune disease include psoriasis, rheumatoid arthritis, systemic lupus erythematosus, Graves' disease, chronic thyroiditis (Hashimoto's disease), type I diabetes, vasculitis (e.g., anti-neutrophil cytoplasmic antibody (ANCA)-associated vasculitis), Addison's disease, polymyositis, Sjogren's syndrome, systemic sclerosis and glomerulonephritis (e.g., IgA nephropathy).

In the present invention, examples of the cancer include solid cancer (carcinomas and sarcomas) and blood tumors, such as skin cancer (e.g., malignant melanoma, Merkel cell carcinoma and squamous cell carcinoma), colon cancer (colorectal cancer), breast cancer (including metastatic breast cancer), gastric cancer, leukemia (e.g., acute lymphocytic leukemia, acute myeloid leukemia, and chronic lymphocytic leukemia), malignant lymphoma (e.g., Hodgkin's lymphoma and non-Hodgkin's lymphoma), and multiple myeloma.

In the present invention, examples of the allergic disease include atopic dermatitis, bronchial asthma, allergic rhinitis, hay fever, food allergy and allergic conjunctivitis.

In the present invention, examples of the neurodegenerative disease include Alzheimer's disease.

In the present invention, examples of the infectious disease include Zika fever (Zika virus infection), SARS-CoV-2 infection (so-called novel coronavirus infection), and influenza infection.

In the present invention, examples of the autoinflammatory disease include familial Mediterranean fever, cryopyrin-associated periodic syndrome, TNF receptorassociated periodic syndrome, mevalonate kinase deficiency, Blau syndrome, periodic fever, aphthous stomatitis, pharyngitis and cervical adenitis syndrome.

In the present invention, examples of diseases other than those described above include rare diseases and intractable diseases, such as multiple sclerosis, neuromyelitis optica, myasthenia gravis, pemphigus, and autoimmune hemolytic anemia.

In the present invention, specific examples of the target protein include proteins as indicated in Table 1.

**[Table 1]**

| Table 1: Example of target protein | |
|---|---|
| IL-17A (Interleukin-17A) | IL-17A is a pro-inflammatory cytokine produced from activated Th17 cells, and acts on various cells including epithelial cells, endothelial cells, fibroblasts, etc. to promote inflammation. IL-17A is known to be associated with autoimmune diseases and cancer. Anti-IL-17A antibodies are used as drugs for treating psoriasis vulgaris. |
| IL-23 (Interleukin-23) | IL-23 is an inflammatory cytokine secreted mainly by activated dendritic cells, macrophages, or monocytes. It is known that IL-23 is associated with autoimmune diseases and cancer, and that the IL-23/IL-17 pathway is associated with the autoimmune disease psoriasis. Anti-IL-23 antibodies are used as drugs for treating psoriasis vulgaris. |
| IL-1β (Interleukin-1β) | IL-1β is produced as a precursor protein by activated macrophages and is processed to its active form by proteolysis by caspase 1. IL-1β induces expression of IL-17, IL-21, and IL-22 along with IL-23. Increased production of IL-1β is known to be associated with many autoimmune diseases. Anti-IL-1β antibodies are used as drugs for treating cryopyrin-associated periodic syndrome (e.g., canakinumab). |
| IL-13 (Interleukin-13) | IL-13 is an immunoregulatory cytokine produced by activated T cells, which downregulates macrophage activity, thereby suppressing production of pro-inflammatory cytokines and chemokines. IL-13 is known as a cytokine associated with allergic diseases and asthma. In clinical trials, anti-IL-13 antibodies have shown efficacy against atopic dermatitis. |
| IL-13R (Interleukin-13 receptor) | IL-13R is a type I cytokine receptor that binds to IL-13. In clinical trials, anti-IL-13R antibodies have shown efficacy against atopic dermatitis. |
| IL-4Rα (Interleukin-4 receptor α-subunit) | IL-4Rα is a receptor that binds to IL-4. Anti-IL-4Rα antibodies are used as drugs for treating atopic dermatitis. |
| TNF-α (Tumor necrosis factor) | TNF-α is a cytokine produced mainly by macrophages. TNF-α is known to be associated with rheumatoid arthritis, psoriasis, diabetes, etc. Anti-TNF-α antibodies are used as drugs for treating rheumatoid arthritis, psoriasis, Crohn's disease, etc. |

**Table 1: Example of target protein (Continuation 1)**

| | |
|---|---|
| IL-6 (Interleukin-6) | IL-6 is a lectin produced by T cells and macrophages, and is a cytokine that controls humoral immunity. Anti-IL-6 antibodies are used as drugs for treating rheumatoid arthritis. |
| IL-6R (Interleukin-6 receptor) | IL-6R is a receptor that binds to IL-6. Anti-IL-6R antibodies are used as drugs for treating rheumatoid arthritis (e.g., tocilizumab). |
| IgE (Immunoglobulin E) | IgE is a type of immunoglobulin and is known to be associated with allergic diseases, atopic diseases, parasitic infections, etc. Anti-IgE antibodies are used as drugs for treating bronchial asthma. |
| IL-5 (Interleukin-5) | IL-5 is a cytokine that controls humoral immunity, and acts mainly on eosinophils to induce their differentiation and proliferation. Anti-IL-5 antibodies are used as drugs for treating bronchial asthma (e.g., mepolizumab). |
| IL-5Rα (Interleukin-5 receptor α) | IL-5Rα is a receptor that binds to IL-5. Anti-IL-5Rα antibodies are used as drugs for treating bronchial asthma (e.g., benralizumab). |
| FcRn (neonatal FC receptor) | FcRn binds to the Fc region of IgG and contributes to recycling of IgG into plasma. Anti-FcRn antibodies are used as drugs for treating myasthenia gravis (e.g., efgartigimod alfa). |
| PD-1 (Programmed cell death 1) | PD-1 is a receptor expressed on the surface of activated T cells. Anti-PD-1 antibodies are used as drugs for treating cancer (e.g., nivolumab and pembrolizumab). |
| PD-L1 (Programmed cell death ligand 1) | PD-L1 is expressed on the surface of antigen presenting cells, vascular endothelium, etc., and plays a role in suppressing T cells to suppress selfattacks. Anti-PD-L1 antibodies are used as drugs for treating cancer (e.g., avelumab, atezolizumab, and durvalumab). |
| CTLA-4 (Cytotoxic T lymphocyte antigen 4) | CTLA-4 is an immune checkpoint receptor expressed on the surface of activated T cells. Anti-CTLA-4 antibodies are used as drugs for treating cancer (e.g., ipilimumab). |
| HER2 (Human epidermal growth factor receptor type 2) | HER2 is a glycoprotein expressed on the cell surface and is a receptor tyrosine kinase. Anti-HER2 antibodies are used as drugs for treating cancer (e.g., trastuzumab and pertuzumab). |
| EGFR (Epidermal growth factor receptor) | EGFR is a receptor tyrosine kinase,, which recognizes an epidermal growth factor (EGF) and is responsible for signaling. Anti-EGFR antibodies are used as drugs for treating cancer (e.g., cetuximab and panitumumab). |

**Table 1: Example of target protein (Continuation 2)**

| | |
|---|---|
| CD22 | CD22 is a transmembrane glycoprotein of the immunoglobulin superfamily and is a membrane antigen specific for mature B cells. Anti-CD22 antibodies are used as drugs for treating cancer (e.g., inotuzumab ozogamicin). |
| CD20 | CD20 is a transmembrane phosphorylated protein and a B cell surface molecule. CD20 functions as a calcium-permeable cation channel and is involved in adjusting activation and proliferation of B cells. Anti-CD20 antibodies are used as drugs for treating cancer (e.g., rituximab). |
| RANKL (Receptor activator of nuclear factor-kappa B ligand) | RANKL is a transmembrane protein that belongs to the TNF superfamily. RANKL is highly expressed in osteoblasts and bone cells, and is known as a ligand molecule that stimulates differentiation and maturation of osteoclasts. Anti-RANKL antibodies are used as drugs for treating osteoporosis (e.g., denosumab). |
| C5 | C5 is a type of complement. Anti-C5 antibodies are used as drugs for treating paroxysmal nocturnal hemoglobinuria and atypical hemolytic uremic syndrome (e.g., eculizumab). |
| IFNγ (Interferon γ) | IFNy is a cytokine secreted mainly from T cells and NK cells, and has an action of activating macrophages. |
| Amyloid β | Amyloid β is a peptide that is deposited in amyloid plaques found in the brains of patients with Alzheimer's disease, and is thought to be centrally associated with the pathology of Alzheimer's disease. In clinical trials, anti-amyloid β antibodies have shown efficacy against Alzheimer's disease. |
| RS virus antigen protein | Examples of RS virus antigen proteins include F protein and G protein. Anti-RS virus antigen antibodies are used as drugs for preventing RS virus infection (e.g., palivizumab). |
| Zika virus antigen protein | Examples of Zika virus antigen protein include capsid protein. |
| SARS-CoV-2 (SARS coronavirus 2) antigen protein | Examples of SARS-CoV-2 antigen protein include spike protein (S protein) of SARS-CoV-2. Anti-SARS-CoV-2 antigen antibodies are used as drugs for treating SARS-CoV-2 infection (so-called novel coronavirus infection). |
| Influenza antigen protein | Examples of influenza virus antigen protein include spike protein (S protein) of influenza virus. Anti-influenza virus antigen antibodies are used as drugs for treating influenza virus infection. |

In the present invention, one or more B cell epitopes of the target protein can be arbitrarily selected as the B cell epitope to be combined depending on the purpose. When two or more B cell epitopes are selected, for example, two or more, three or more, four or more, five or more, six or more, seven or more, eight or more, or nine or more B cell epitopes can be selected. When the target protein is a protein that causes a disease and/or a protein that is produced due to a disease, the B cell epitope may be selected from the viewpoint of preventing or treating the disease.

In the present invention, the T cell epitope and B cell epitope to be combined can be selected from any types of T cell epitopes and B cell epitopes, respectively, and can be selected from, for example, the T cell epitopes and B cell epitopes exemplified above.

The nucleic acid construct or protein complex of the present invention can be designed and produced by known molecular biological methods based on the combination of selected T cell epitopes and B cell epitopes. Specifically, the protein complex can be designed and produced as a fusion protein comprising T cell epitopes and B cell epitopes as components.

The nucleic acid construct of the present invention can be designed and produced as a single polynucleotide encoding a protein complex, i.e., a fusion protein comprising T cell epitopes and B cell epitopes as components. Therefore, in this sense, the nucleic acid construct of the present invention can be rephrased as "nucleic acid construct encoding a protein complex that induces production of an antibody against a target protein." In the nucleic acid construct of the present invention, from the viewpoint of effectively inducing antibody production in the living body of the subject, the order of the polynucleotide sequences encoding the T cell epitope and the B cell epitope, respectively, can be appropriately adjusted, and a sequence that controls expression or a linker sequence can be inserted between the respective epitopes. Furthermore, the B cell epitope to be combined with the T cell epitopes may be composed of the entire target protein recognized by an antigen-specific B cell, or may be composed of a part of the target protein including an antigen moiety (e.g., a domain).

The nucleic acid construct of the present invention is composed of polynucleotides, and the polynucleotides can be polynucleotides encoding two or more T cell epitopes and one or more B cell epitopes of a target protein. The polynucleotides include DNA and RNA, as well as modified products thereof and artificial nucleic acids. From the viewpoint of using the nucleic acid construct as an active ingredient of a vaccine, RNA is preferable.

The protein complex of the present invention is configured as a fusion protein comprising, as components, two or more T cell epitopes and one or more B cell epitopes of the target protein. The protein complex may be modified as long as the T cell epitopes and the B cell epitopes each function as epitopes.

The nucleic acid construct or protein complex of the present invention can induce production of an antibody against a target protein. Namely, by administering the nucleic acid construct or protein complex of the present invention to a subject, antibody production can be induced in the body of the subject.

Without being bound by the following theory, the mechanism of action from preparation of a nucleic acid construct or protein complex, to administration of the prepared nucleic acid construct or protein complex to a subject and induction of antibody production in the body of the subject will be described.

### [I. Case of nucleic acid construct]

(i) A nucleic acid construct to be administered to a subject is prepared by a known molecular biological method based on the nucleic acid construct designed by the design method of the present invention.
(ii) The prepared nucleic acid construct is loaded onto a carrier (a lipid membrane such as a liposome) and administered to the subject by a method such as injection.
(iii) The administered nucleic acid construct is translated in the living body of the subject to produce a protein (a fusion protein comprising T cell epitopes and B cell epitopes as components).
(iv) The produced fusion protein is recognized by an antigen-specific B cell in the living body of the subject, taken up into the cell, and digested.
(v) Within the B cell, the T cell epitope forms a complex with an MHC class II molecule, and presents the complex on the surface of the B cell.
(vi) A CD4-positive T cell recognizes and binds to a complex of the T cell epitope and the MHC class II molecule via a T cell receptor (TCR), and is activated.
(vii) The activated CD4-positive T cell clonally proliferates and promotes proliferation and differentiation of the B cell.
(viii) The B cell is activated and clonally proliferates, thereby producing a large amount of the antibody of interest.

### [II. Case of protein complex]

(i) Based on the protein complex designed by the design method of the present invention, a protein complex (a fusion protein comprising T cell epitopes and B cell epitopes as components) to be administered to a subject is prepared by a known molecular biological method.
(ii) The prepared protein complex is administered to the subject by a method such as injection.
(iii) The administered protein complex is recognized by an antigen-specific B cell in the living body of the subject, taken up into the cell, and digested.
(iv) Within the B cell, the T cell epitope forms a complex with an MHC class II molecule, and presents the complex on the surface of the B cell.
(v) A CD4-positive T cell recognizes and binds to a complex of the T cell epitope and the MHC class II molecule via a T cell receptor (TCR), and is activated.
(vi) The activated CD4-positive T cell clonally proliferates and promotes proliferation and differentiation of the B cell.
(vii) The B cell is activated and clonally proliferates, thereby producing a large amount of the antibody of interest.

The nucleic acid construct of the present invention can induce production of an antibody against a target protein in a subject, and can therefore be used to prevent or treat a disease associated with the target protein, and also to reduce the risk of suffering from the disease associated with the target protein. That is, according to the present invention, there is provided an agent for preventing or treating a disease, which comprises the nucleic acid construct of the present invention as an active ingredient. Also, according to the present invention, there is provided an agent for reducing the risk of suffering from a disease, which comprises the nucleic acid construct of the present invention as an active ingredient. The agents for preventing and treating a disease of the present invention can be administered to a subject having a disease or a subject likely to have a disease. The agent for reducing the risk of the present invention can be administered to a subject likely to have a disease.

When the nucleic acid construct of the present invention is administered to a subject, the route of administration is not particularly limited as long as the effect for treating or preventing the target disease or the effect for reducing the risk of suffering from the target disease. For example, parenteral administration can be selected. Non-limiting examples of the parenteral administration include intravenous administration, intramuscular administration, subcutaneous administration, topical administration, intraperitoneal administration, and intranasal administration.

As an agent for parenteral administration, an appropriate dosage form can be selected depending on the specific administration route, and examples thereof include injections. The agent for parenteral administration can be in the form of an aqueous or non-aqueous isotonic sterile solution or suspension. Any of the preparations can be formulated using a pharmaceutically acceptable carrier by a technique commonly used in the art (e.g., the known method described in the General Rules for Preparations of the Japanese Pharmacopoeia 18^{th} edition). That is, according to the present invention, there is provided a pharmaceutical composition comprising the nucleic acid construct of the present invention and a pharmaceutically acceptable carrier. The pharmaceutically acceptable carrier includes excipients, binders, diluents, additives, perfumes, buffers, thickeners, colorants, stabilizers, emulsifiers, dispersants, suspending agents, preservatives, and the like. The agent of the present invention can be formulated, for example, by using the nucleic acid construct of the present invention in the form of a lipid nanoparticle (LNP), and examples of lipids constituting the LNP include pH-responsive lipids (e.g., DLin-MC3-DMA, DLin-DMA, and DLin-KC2-DMA), PEGylated lipids (e.g., PEG-DMG, PEG-DSG, and PEG-DPG), cholesterol, and neutral phospholipids.

The dose of the nucleic acid construct of the present invention can be determined depending on the sex, age, and body weight of the subject, symptoms, dosage form, administration route, and the like. In the present invention, when the nucleic acid construct is administered for the purpose of treating or preventing a disease, the daily dose for adults can be determined in the range of, for example, 0.01 µg to 100 mg, but is not limited thereto. In the present invention, the above-described dose of the active ingredient may be administered once a day, or may be divided into 2 to 4 portions for administration. Also, in the present invention, the above-described dose of the active ingredient may be administered multiple times, initially at intervals of once every one, two, three or four weeks, and then once every one, two, three, four, five, or six months or one year.

In the present invention, the subject includes humans and non-human animals, and the non-human animals are preferably non-human mammals (e.g., mice, rats, cows, pigs, horses, and monkeys).

### <<Design method>>

According to another aspect of the present invention, there is provided a method for designing a nucleic acid construct or protein complex. According to the design method of the present invention, it is possible to design a nucleic acid construct or protein complex that induces production of an antibody against a target protein.

The design method of the present invention comprises the step of combining two or more T cell epitopes and one or more B cell epitopes of the target protein.

The design method of the present invention can comprise the steps of: (X1) evaluating that T cell epitopes have an ability to bind to an MHC class II molecule using an MHC binding prediction tool; and (X2) selecting T cell epitopes having a high ability to bind to an MHC class II molecule based on the evaluation results of the step (X1). In the design method of the present invention, the above steps (X1) and (X2) can be carried out prior to the step of combining two or more T cell epitopes and one or more B cell epitopes of the target protein. The design method of the present invention may also comprise the step of preliminarily screening T cell epitopes prior to the above steps (X1) and (X2). This preliminary screening can be carried out using AI (Artificial Intelligence) and a predictive algorithm.

The design method of the present invention can be implemented according to the descriptions about the nucleic acid construct of the present invention, in addition to the above description.

### <<Agent for preventing or treating disease and method for producing same>>

According to another aspect of the present invention, there is provided an agent for preventing or treating a disease. The agent of the present invention is characterized by comprising the nucleic acid construct or protein complex of the present invention as an active ingredient.

In the agent of the present invention, the target protein can be a protein that causes a disease or a protein that is produced due to a disease. In the production method of the present invention, the target protein can also be a protein produced in a subject's body.

The agent for preventing a disease of the present invention can typically be used as a vaccine. Specifically, the agent for preventing a disease of the present invention can be used for the purpose of inducing production of an antibody against the target protein in the living body of a subject through administration to the subject, thereby preventing the subject from suffering from or developing a disease, or, if the subject has suffered from or developed the disease, reducing the severity of the disease. Examples of the agent for preventing a disease of the present invention include mRNA vaccines, gene recombinant protein vaccines, and peptide vaccines. From the viewpoint of production cost, an mRNA vaccine is preferable.

The agent for treating a disease of the present invention can typically be used as an alternative to antibody drugs. Specifically, the agent for treating a disease of the present invention can be used for the purpose of inducing production of an antibody against a target protein in the living body of a subject through administration to the subject, thereby treating the disease which the subject has suffered from or developed.

According to another aspect of the present invention, there is provided a method for producing the agent for preventing or treating a disease of the present invention. The production method of the present invention comprises: (P) designing a nucleic acid construct or protein complex that induces production of an antibody against a target protein; and (Q) preparing the nucleic acid construct or protein complex designed in the step (P).

The design of the nucleic acid construct or protein complex in the step (P) can be implemented according to the descriptions about the design method of the present invention.

The preparation of the nucleic acid construct or protein complex in the step (Q) can be carried out by a known method. For example, without any limitation, the nucleic acid construct can be prepared by chemical synthesis or in vitro transcription synthesis of oligonucleotides, and the protein complex can be prepared using an expression vector incorporated with the nucleic acid construct.

The agent for preventing or treating a disease and method for producing the agent according to the present invention can be implemented according to the descriptions about the nucleic acid construct of the present invention and the design method of the present invention, in addition to the above description.

According to the present invention, there is provided a method for preventing or treating a disease, the method comprising administering a therapeutically or prophylactically effective amount of a nucleic acid construct or a composition comprising the nucleic acid construct to a subject in need thereof. Also, according to the present invention, there is provided a method for reducing the risk of suffering from a disease, the method comprising administering an effective amount of a nucleic acid construct or a composition comprising the nucleic acid construct to a subject in need thereof. The methods of the present invention can be implemented according to the descriptions about the nucleic acid construct of the present invention and the agents of the present invention.

Also, according to the present invention, there is provided use of the nucleic acid construct of the present invention for production of an agent for preventing or treating a disease, or as an agent for preventing or treating a disease. Also, according to the present invention, there is provided use of the nucleic acid construct of the present invention for production of an agent for reducing the risk of suffering from a disease, or as an agent for reducing the risk of suffering from a disease. The methods of the present invention can be implemented according to the descriptions about the nucleic acid construct of the present invention and the agents of the present invention.

### EXAMPLES

The present invention will be described in more detail by way of the following examples, but is not limited to these examples.

### Example 1: Design of amino acid sequence of T cell epitope (1)

In Example 1, the amino sequences of the mouse T cell epitopes indicated in Table 2 were designed. In addition, the MHC class II binding abilities of the amino acid sequences of the T cell epitopes indicated in Table 2 were analyzed using an IEDB analysis resource.

**[Table 2]**

| Table 2: Amino acid sequence of T cell epitope | |
|---|---|
| SEQ ID NO: 83 | FASVYAWNRKRIVTQQL |
| SEQ ID NO: 84 | YNYLYRLFRKSNLK |
| SEQ ID NO: 85 | QLIRAAEIRASANLAAGG |

The results were as indicated in Tables 3 to 5. For the T cell epitopes indicated in these tables, a lower numerical value for the MHC class II binding ability indicates a higher binding ability, and it was suggested that the lower the numerical value for the MHC class II binding ability, the higher the antibody production inducing activity. The results in Tables 3 to 5 suggested that these T cell epitopes have a high MHC class II binding ability and a high antibody production inducing activity in Balb/c mice.

**[Table 3]**

| Table 3: MHC class Il binding ability and antibody production inducing activity | | | |
|---|---|---|---|
| Amino acid sequence (SEQ ID of T cell epitope NO: 1) | | MHC class II binding ability | Antibody production inducing activity |
| Balb/c mouse | H2-IE^{d} restrictive | 0.05-0.08 | Very high |
| C57BL/6 mouse | H2-IA^{b} restrictive | > 9.0 | None |

**[Table 4]**

| Table 4: MHC class 11 binding ability and antibody production inducing activity | | | |
|---|---|---|---|
| Amino acid sequence of T cell epitope (SEQ ID NO: 2) | | MHC class II binding ability | Antibody production inducing activity |
| Balb/c mouse | H2-IE^{d} restrictive | 0.16 | Very high |
| C57BL/6 mouse | H2-IA^{b} restrictive | > 3.0 | None |

**[Table 5]**

| Table 5: MHC class II binding ability and antibody production inducing activity | | | |
|---|---|---|---|
| Amino acid sequence of T cell epitope (SEQ ID NO: 3) | | MHC class II binding ability | Antibody production inducing activity |
| Balb/c mouse | H2-IA^{d} restrictive | 0.55 | High |
| C57BL/6 mouse | H2-IA^{b} restrictive | 2.6 | High |

### Example 2: Design of amino acid sequence of T cell epitope (2)

In Example 2, the amino acid sequences of human T cell epitopes were designed, and the MHC class II binding abilities of the T cell epitopes were analyzed using an IEDB analysis resource.

The results were as indicated in Tables 6 and 7. For the T cell epitopes indicated in these tables, a lower numerical value for the MHC class II binding ability indicates a higher binding ability, and it was suggested that the lower the numerical value for the MHC class II binding ability, the higher the antibody production inducing activity. The results in Tables 6 and 7 suggested that a high MHC class II binding ability and a high antibody production inducing activity were observed in most of both the Japanese and Westerner populations. This indicates that the present nucleic acid construct is pan-MHC compatible, and is applicable to most of humans including the Japanese population.

**[Table 6]**

| | | | |
|---|---|---|---|
| Table 6: MHC class Il Japanese binding ability and antibody production inducing activity in | | | |

| MHC class II (HLA II) | Frequency (%)(*) | MHC class II (HLA (II) binding ability | Antibody production inducing activity |
|---|---|---|---|
| DRB1*01:01 | 5.8 | 0.01 | Very high |
| DRB1*04:03 | 3.1 | 0.17 | Very high |
| DRB1*04:05 | 13.5 | 0.66 | High |
| DRB1*04:06 | 3.4 | 0.17 | Very high |
| DRB1*08:02 | 4.2 | 1.70 | Moderate |
| DRB1*08:03 | 8.3 | 0.01 | Very high |
| DRB1*09:01 | 14.3 | 0.28 | High |
| DRB1*12:01 | 3.8 | 1.18 | Moderate |
| DRB1*13:02 | 5.9 | 0.01 | Very high |
| DRB1*14:54 | 3.3 | 0.29 | Very high |
| DRB1*15:01 | 7.8 | 0.53 | High |
| DRB1*15:02 | 10.7 | 0.23 | Very high |
| DRB1*11:01 | 2.5 | 0.22 | Very high |

| | | | |
|---|---|---|---|
| *Frequency is quoted from Ikeda N, et al., Tissue Antigens; 85(4): 252-9 (2015). | | | |

**[Table 7]**

| Table 7: MHC class II binding ability and antigen production inducing activity in Westerner | | | |
|---|---|---|---|
| MHC class II (HLA II) | Frequency (rank) ( 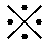 ) | MHC class II binding ability | Antibody production inducing activity |
| DRB1*07:01 | 1 | 0.33 | High |
| DRB1*03:01 | 2 | 0.51 | High |
| DRB1*04:01 | 3 | 0.73 | High |
| DRB1*15:01 | 4 | 0.53 | High |

| | | | |
|---|---|---|---|
| *Frequency is quoted from Mack SJ, et al., Tissue Antigens; 73(1):17-32 (2009). | | | |

### Example 3: Study on ability of nucleic acid construct encoding T cell epitope to induce antibody (1)

In Example 3, nucleic acid constructs comprising a polynucleotide encoding the T cell epitopes designed in Example 1 were used to study the ability to induce antibody production in mice.

### (1) Method

### A. Preparation of nucleic acid construct (mRNA vaccine)

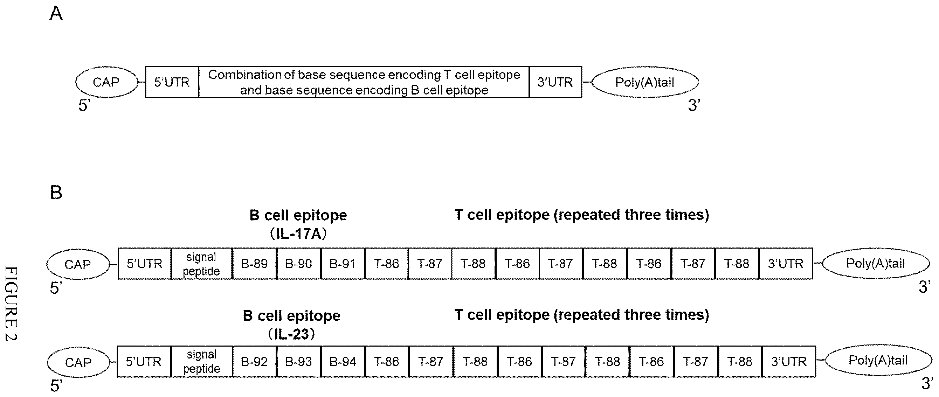

A nucleic acid construct (IL-17A nucleic acid construct) containing a base sequence in which each of the three base sequences encoding T cell epitopes indicated in Table 8 (with T replaced by Ψ) was repeated three times and the three base sequences encoding B cell epitopes of mouse IL-17A indicated in Table 9 (with T replaced by Ψ), and a nucleic acid construct (IL-23 nucleic acid construct) containing a base sequence in which each of the three base sequences encoding T cell epitopes indicated in Table 8 (with T replaced by Ψ) was repeated three times and the three base sequences encoding B cell epitopes of mouse IL-23 indicated in Table 10 (with T replaced by Ψ) were prepared according to a conventional method (as for the compositions of the nucleic acid constructs, see also FIGS. 2A and 2B). Ψ represents 1-methyl-3-pseudouridylyl.

**[Table 8]**

| Table 8: Base sequence encoding T cell epitope* | |
|---|---|
| SEQ ID NO: 86 | TTCGCCTCTGTGTACGCCTGGAACCGGAAGCGGATCGTGA CCCAGCAGCTG |
| SEQ ID NO: 87 | TACAATTACCTGTACCGGCTGTTCCGGAAGTCCAATCTGA AG |
| SEQ ID NO: 88 | CAGCTGATCAGAGCCGCCGAGATTAGAGCCTCTGCCAATC |
| | TGGCCGCCGG CGGC |

| | |
|---|---|
| *The base sequences of SEQ ID NOs: 86 to 88 correspond to the amino acid sequences of the T cell epitopes of SEQ ID NOs: 83 to 85, respectively, indicated in Table 2. | |

**[Table 9]**

| Table 9: Base sequence encoding B cell epitope of IL-17A | |
|---|---|
| SEQ ID NO: 89 | ATGAGCCCCGGCAGAGCCTCCAGCGTGAGCCTGATGCTG CTGCTGCTGCTGAGCCTGGCCGCCACCGTGAAGGCCGCC GCCATCATCCCCCAGTCCAGCGCCTGCCCCAACACCGAG GCCAAG |
| SEQ ID NO: 90 | GACTTCCTGCAGAACGTGAAGGTGAACCTGAAGGTGTTC AACAGCCTGGGCGCCAAGGTGTCCAGCAGAAGACCCAG CGACTACCTGAACAGAAGCACCAGCCCCTGGACCCTGCA CAGAAACGAGGACCCCGACAGATACCCCAGCGTGATCTG GGAGGCCCAGTGCAGACACCAGAGATGCGTG |
| SEQ ID NO: 91 | AACGCCGAGGGCAAGCTGGACCACCACATGAACAGCGT GCTGATCCAGCAGGAGATCCTGGTGCTGAAGAGAGAGCC CGAGAGCTGCCCCTTCACCTTCAGAGTGGAGAAGATGCT GGTGGGCGTGGGCTGCACCTGCGTGGCCAGCATCGTGAG ACAGGCCGCC |

**[Table 10]**

| Table 10: | Base sequence encoding B cell epitope of IL-23 |
|---|---|
| SEQ ID NO: 92 | ATGCTGGACTGCAGAGCCGTGATCATGCTGTGGCTGCTGC CCTGGGTGACCCAGGGCCTGGCCGTGCCCAGATCCAGCA GCCCCGACTGGGCCCAGTGCCAGCAGCTGAGCAGAAACC TGTGCATGCTGGCCTGGAACGCCCACGCCCCCGCCGGCCA CATGAACCTGCTGAGAGAGGAGGAGGACGAG |
| SEQ ID NO: 93 | GAGACCAAGAACAACGTGCCCAGAATCCAGTGCGAGGAC GGCTGCGACCCCCAGGGCCTGAAGGACAACAGCCAGTTC TGCCTGCAGAGAATCAGACAGGGCCTGGCCTTCTACAAGC ACCTGCTGGACAGCGACATCTTCAAGGGCGAGCCCGCCCT GCTGCCCGACAGCCCCATGGAGCAGCTGCACACCAGC |
| SEQ ID NO: 94 | CTGCTGGGCCTGAGCCAGCTGCTGCAGCCCGAGGACCAC CCCAGAGAGACCCAGCAGATGCCCAGCCTGTCCAGCAGC CAGCAGTGGCAGAGACCCCTGCTGAGAAGCAAGATCCTG AGAAGCCTGCAGGCCTTCCTGGCCATCGCCGCCAGAGTGT TCGCCCACGGCGCCGCCACCCTGACCGAGCCCCTGGTGCC C ACCGCC |

### B. Measurement of antibody titer

The IL-17A nucleic acid construct and IL-23 nucleic acid construct prepared in A above were each administered in an amount of 10 µg to Balb/c mice at intervals of two weeks. Then, for sera (200-fold diluted) collected before the start of administration (week 0), and 2, 4, 6, 8, and 10 weeks after the start of administration, the antibody titers specific for mouse IL-17A and mouse IL-23 were measured by the ELISA method using recombinant native proteins of mouse IL-17A and mouse IL-23, respectively. Table 11 indicates procedures for measuring the antibody titers.

**[Table 11]**

| Table 11. Procedure for measuring antibody titer |
|---|
| <Reagent and the like> |
| -96-well microplate: MAXISORP (Nunc) |
| -Substrate: TMB |
| -Solid phasing buffer: 0.1 M carbonate butter, pH 9.5 (IBL) |
| -Blocking buffer: 0.1% BSA, 0.05% NaN3 in D-PBS(-) (IBL) |
| -Dilution buffer: 1% BSA, 0.05% Tween-20 in D-PBS(-) (IBL) |
| -Labeled antibody: AntiMouse IgG (Goat)-HRP (Southern Biotech) |
| -Labeled antibody dilution buffer: 1% BSA, 0.05% Tween-20 in D-PBS(-) (IBL) |
| -Washing buffer: 0.05% Tween-20 in 0.1 M phosphate buffer (IBL) |
| -Reaction stopping solution: 1 N-H₂SO₄ (IBL) |
| -Absorbance measurement device for multiplate: E. Max (Molecular Devices) |
| -Antigen: Recombinant mouse IL-17A protein (Active), ab281805(abcam), |
| Recombinant mouse IL-23 protein (Active), ab259423 (abcam) |
| <Procedures> |
| 1. Solid phasing: Each antigen prepared with a solid phasing buffer was incubated at 50 ng/well (50 µL/well) at 4°C for 16 hours or more. |
| 2. Washing: After addition of D-PBS(-) at 200 µL/well, a washing solution was removed. |
| 3. Blocking: A blocking buffer was added at 200 µL/well, followed by incubation overnight at 4°C. |
| 4. Washing: After addition of a washing buffer at 200 µL/well, a washing solution was |
| removed. |
| 5. Sample: After 200-fold dilution of mouse serum with a dilution buffer, the sample was added at 50 µL/well, followed by incubation at 37°C for 30 minutes. |
| 6. Washing: After addition of a washing buffer at 200 µL/well, a washing solution was removed. This procedure was repeated two more times (three times in total). |
| 7. Labeled antibody: Anti-Mouse IgG-HRP was added at 50 µL/well (diluted with a dilution buffer), followed by incubation at 37°C for 30 minutes. |
| 8. Washing: After addition of a washing buffer at 200 µL/well, a washing solution was removed. This procedure was repeated three more times (four times in total). |
| 9. Color development: A substrate solution was added at 100 µL/well, and the mixture was reacted at room temperature for 30 minutes (light shielded). |
| 10. Reaction stopping: A reaction stopping solution was added at 100 µL/well to stop the reaction. |
| 11. Absorbance measurement: The absorbance in each well was measured using an absorbance measuring device for multiplates (wavelength: 450 nm). |

### (2) Result

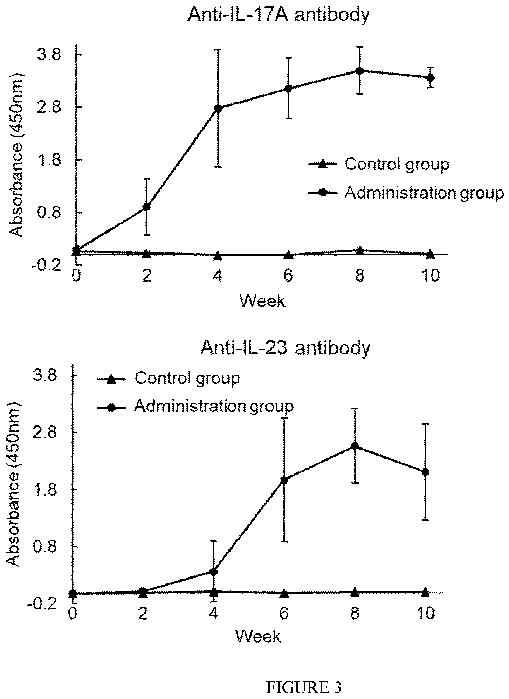

The results were as shown in FIG. 3. In the mice administered with the nucleic acid construct (mRNA vaccine) (administration group), rises in antibody titers against IL-17A and IL-23 were observed after the start of administration. In contrast, in the mice not administered with the mRNA vaccine (control group), no rises in antibody titers against IL-17A and IL-23 were observed. These results demonstrated that the nucleic acid constructs (mRNA vaccines) comprising the polynucleotide encoding the T cell epitopes designed in Example 1 actually induced the production of antibodies against IL-17A and IL-23 in the mice.

### Example 4: Study on ability of nucleic acid construct encoding T cell epitope to induce antibody (2)

In Example 4, nucleic acid constructs comprising a polynucleotide encoding the T cell epitopes designed in Example 1 were used to study subclasses of antibodies, the production of which was induced in mice.

### (1) Method

### A. Nucleic acid construct (mRNA vaccine)

In the same manner as in Example 3, (1), A, an IL-17A nucleic acid construct and an IL-23 nucleic acid construct were prepared as mRNA vaccines.

### B. Measurement of antibody titer

The IL-17A nucleic acid construct and IL-23 nucleic acid construct prepared in A above were each administered in an amount of 10 µg to Balb/c mice at intervals of two weeks. For sera (200-fold diluted, 800-fold diluted, 3200-fold diluted, and 12800-fold diluted) 6 weeks after the start of administration, the antibody titer of each of the subclass antibodies (IgG1, IgG2a, IgG2b, and IgG3) against mouse IL-17A and mouse IL-23 was measured by the ELISA method. Table 12 indicates procedures for measuring the antibody titers.

**[Table 12]**

| Table 12. Procedure for measuring antibody titer | |
|---|---|
| <Reagent and the like> | |
| -96-well microplate: MAXISORP (Nunc) | |
| -Substrate: TMB | |
| -Solid phasing buffer: 0.1 M carbonate butter, pH 9.5 (IBL) | |
| -Blocking buffer: 0.1% BSA, 0.05% NaN3 in D-PBS(-) (IBL) | |
| -Dilution buffer: 1% BSA, 0.05% Tween-20 in D-PBS(-) (IBL) | |
| -Labeled antibody: AntiMouse IgG (Goat)-I-A RP (Southern Biotech) | |
| -Labeled antibody dilution buffer: 1% BSA, 0.05% Tween-20 in D-PBS(-) (IBL) | |
| -Washing buffer: 0.05% Tween-20 in 0.1 M phosphate buffer (IBL) | |
| -Reaction stopping solution: 1 N-H₂SO₄ (IBL) | |
| -Absorbance measurement device for multiplate: E. Max (Molecular Devices) | |
| -Antigen: Recombinant mouse IL-17A protein (Active), ab281805(abcam), | |
| Recombinant mouse IL-23 protein (Active), ab259423 (abcam) | |
| <Procedures> | |
| 1. Solid phasing: Each antigen prepared with a solid phasing buffer was incubated at | |
| 50 ng/well (50 µL/well) at 4°C for 16 hours or more. | |
| 2. Washing: After addition of D-PBS (-) at 200 µL/well, a washing solution was removed. | |
| 3. Blocking: A blocking buffer was added at 200 µL/well, followed by incubation overnight at 4°C. | |
| 4. Washing: After addition of a washing buffer at 200 µL/well, a washing solution was removed. | |
| 5. Sample: After 200-fold dilution of mouse serum with a dilution buffer, the sample was added at 50 µL/well, followed by incubation at 37°C for 30 minutes. | |
| 6. Washing: After addition of a washing buffer at 200 µL/well, a washing solution was removed. This procedure was repeated two more times (three times in total). | |
| 7. Antibody against subclass antibody: Each antibody (Anti-Mouse IgG1 Rabbit IgG, Anti-Mouse IgG2a Rabbit IgG, Anti-Mouse IgG2b Rabbit IgG, and Anti-Mouse IgG3 Rabbit IgG) was added at 1 µg/mL (50 µL/well). Incubation was performed at 37°C for 30 minutes. | |
| 8. Washing: After addition of a washing buffer at 200 µL/well, a washing solution was removed. This procedure was repeated two more times (three times in total). | |
| 9. Labeled antibody: Anti-Mouse IgG-HRP was added at 50 µL/well (diluted with a dilution buffer), followed by incubation at 37°C for 30 minutes. | |
| 10. Washing: After addition of a washing buffer at 200 µL/well, a washing solution was removed. This procedure was repeated three more times (four times in total). | |
| 11. Color development: A substrate solution was added at 100 µL/well, and the mixture was reacted at room temperature for 30 minutes (light shielded). | |
| 12. Reaction stopping: A reaction stopping solution was added at 100 µL/well to stop the reaction. | |
| 13. Absorbance measurement: The absorbance in each well was measured using an absorbance measuring device for multiplates (wavelength: 450 nm). | |

### (2) Result

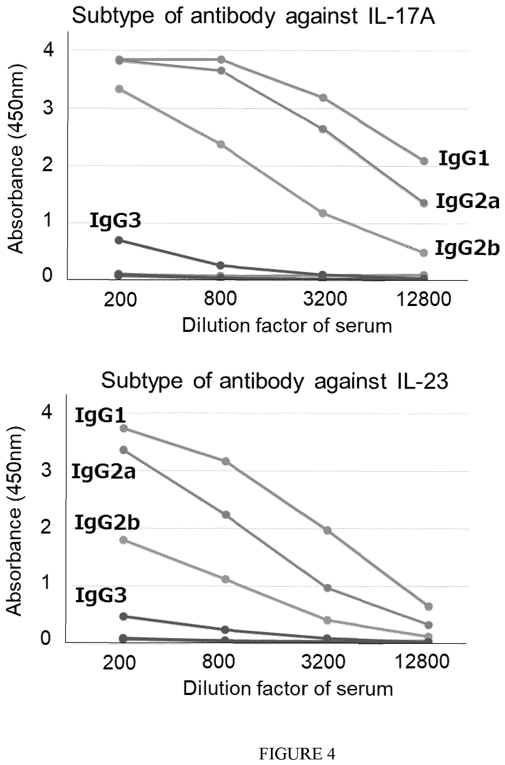

The results were as shown in FIG. 4. In the mice administered with the nucleic acid construct (mRNA vaccine) (administration group), the antibody titers of IgG2a and IgG1, among the subclasses of antibodies against IL-17A and IL-23, 6 weeks after the start of administration, were high. In contrast, in the mice not administered with the mRNA vaccine (control group), the antibody titers of all the subclasses were close to 0. These results demonstrated that the antibodies induced by the nucleic acid constructs (mRNA vaccines) comprising the polynucleotide encoding the T cell epitopes designed in Example 1 were both IgG2a induced by a Th1-type immune response and IgG1 induced by a Th2-type immune response.

### Example 5: Study on ability of nucleic acid construct encoding T cell epitope to induce antibody (3)

In Example 5, nucleic acid constructs comprising a polynucleotide encoding the T cell epitopes designed in Example 1 were used to study the ability to induce antibody production in C57BL/6 mice (H2-IA^{b}) having MHC class II different from that of the Balb/c mice (H2-IE^{d} and H2-IA^{d}) used in Example 3.

### (1) Method

### A. Preparation of nucleic acid construct (mRNA vaccine)

In the same manner as in Example 3, (1), A, an IL-17A nucleic acid construct and an IL-23 nucleic acid construct were prepared as mRNA vaccines.

### B. Measurement of antibody titer

The antibody titers were measured in the same manner as in Example 3, (1), B, except that C57BL/6 mice were used instead of the Balb/c mice.

### (2) Result

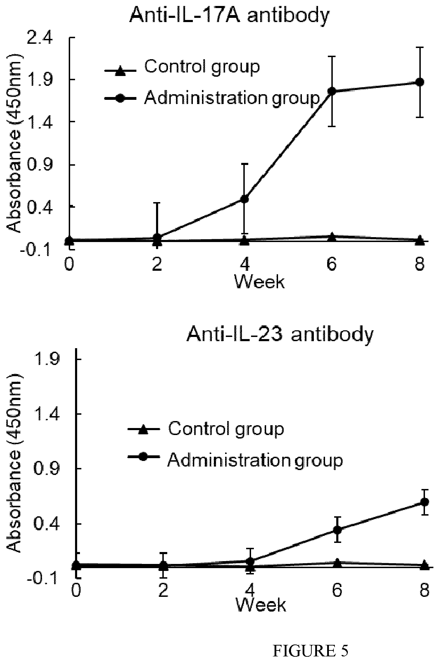

The results were as shown in FIG. 5. In the mice administered with the nucleic acid construct (mRNA vaccine) (administration group), rises in antibody titers against IL-17A and IL-23 were observed after the start of administration. In contrast, in the mice not administered with the mRNA vaccine (control group), no rises in antibody titers against IL-17A and IL-23 were observed. These results demonstrated that the nucleic acid constructs (mRNA vaccines) comprising the polynucleotide encoding the T cell epitopes designed in Example 1 induced the production of the antibodies against IL-17A and IL-23 even in the mice having different MHC class II, and that the nucleic acid construct of the present invention is applicable as a pan-HLA compatible vaccine.

### Example 6: Study on efficacy of antibody induced by nucleic acid construct encoding T cell epitope

In Example 6, the efficacy of antibodies, the production of which was induced by nucleic acid constructs (mRNA vaccines) comprising a polynucleotide encoding the T cell epitopes designed in Example 1, was studied.

### (1) Method

### A. Preparation of nucleic acid construct (mRNA vaccine)

In the same manner as in Example 3, (1), A, an IL-17A nucleic acid construct and an IL-23 nucleic acid construct were prepared as mRNA vaccines.

### B. Antibody induction by mRNA vaccine

Antibody production was induced by administering 10 µg of each of the IL-17A nucleic acid construct and IL-23 nucleic acid construct prepared in A above to Balb/c mice at intervals of two-weeks, and plasma was collected 20 weeks after the start of administration.

### C. Antibody administration test

A comparative study of ear thickness change was made between Balb/c mice (without antibody administration) in which psoriasis was induced by applying 10 mg each of 5% imiquimod (IMQ) cream (BESELNA Cream 5%, Mochida Pharmaceutical Co., Ltd.) to the front and back surfaces of their ears for 5 consecutive days (control group) and mice in which psoriasis was induced by intraperitoneally administering 0.15 mL of the plasma prepared in B above to Balb/c mice, and, immediately after administration, applying 10 mg each of 5% IMQ cream to the front and back surfaces of their ears for 5 consecutive days (administration group). The thicknesses of both ears were measured daily using a digital thickness gauge (OZAKI MFG. CO., LTD.). The inhibition rate (%) (inhibition rate (%) in administration group = (1 - (administration group mice - average value of normal mice)/(average value of IMQ mice - average value of normal mice)) × 100; inhibition rate (%) in control group = (1 - (control group mice - average value of normal mice)/(average value of control group mice - average value of normal mice)) was calculated based on the thicknesses of both ears 5 days after application of IMQ. Photographs of mouse ears were taken after repeated application of IMQ for 6 days. In addition, a change in ear thickness of the normal mice was also measured, and the mouse ears were photographed.

### (2) Result

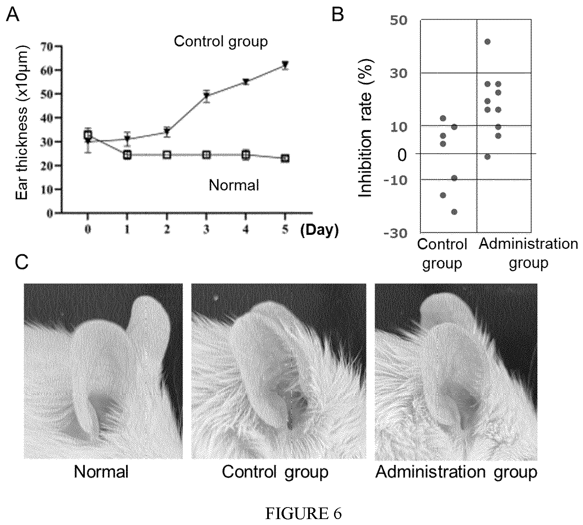

The results were as shown in FIG. 6. In the control group, the ear thickness increased with each passing day (FIG. 6A). Furthermore, the inhibition rate calculated based on the thicknesses of both ears 5 days after application of IMQ was higher in the administration group than in the control group (FIG. 6B). It was observed, also from the photographs in FIG. 6C, that psoriasis-like symptoms (thickness and inflammation) of the ears in the administration group were suppressed as compared with those in the control group. These results demonstrated the efficacy of the antibodies, the production of which was induced by the nucleic acid constructs (mRNA vaccines) comprising the polynucleotide encoding the T cell epitopes designed in Example 1.

### Example 7: Study on efficacy of nucleic acid construct (mRNA vaccine) encoding T cell epitope

In Example 7, the efficacy of nucleic acid constructs (mRNA vaccines) comprising a polynucleotide encoding the T cell epitopes designed in Example 1 was studied.

### (1) Method

### A. Preparation of nucleic acid construct (mRNA vaccine)

In the same manner as in Example 3, (1), A, an IL-17A nucleic acid construct and an IL-23 nucleic acid construct were prepared as mRNA vaccines.

### B. mRNA vaccine administration test

The IL-17A nucleic acid construct and IL-23 nucleic acid construct prepared in A above were each administered twice in an amount of 10 µg to Balb/c mice at intervals of two weeks. Four (4) weeks after the start of administration, a comparative study of ear thickness change was made between mice in which psoriasis was induced by applying 5 mg each of 5% IMQ cream to the front surfaces of the ears of the Balb/c mice for 6 consecutive days (administration group) and Balb/c mice (without administration of the mRNA vaccine) in which psoriasis was similarly induced (control group). The thicknesses of both ears were measured daily using a digital thickness gauge (OZAKI MFG. CO., LTD.). The inhibition rate (%) (inhibition rate (%) in administration group = (1 - (administration group mice - average value of normal mice)/(average value of IMQ mice - average value of normal mice)) × 100; inhibition rate (%) in control group = (1 - (control group mice - average value of normal mice)/(average value of control group mice - average value of normal mice)) was calculated based on the thicknesses of both ears 6 days after application of IMQ. Photographs of mouse ears were taken after repeated application of IMQ for 6 days. In addition, a change in ear thickness of the normal mice was also measured, and the mouse ears were photographed.

### (2) Result

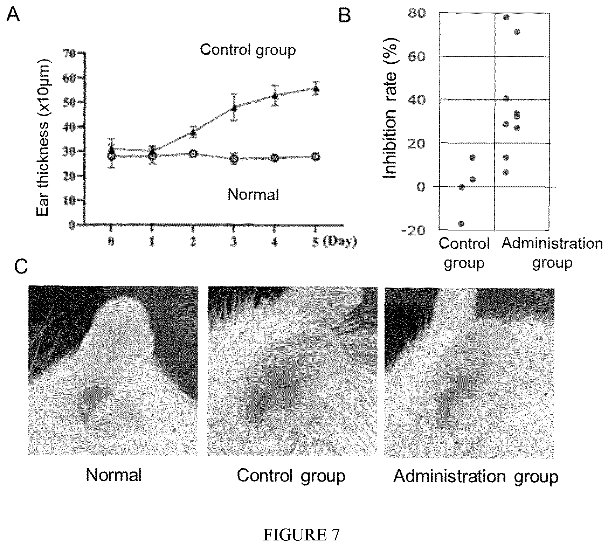

The results were as shown in FIG. 7. In the control group, the ear thickness increased with each passing day (FIG. 7A). The inhibition rate calculated based on the thicknesses of both ears 6 days after application of IMQ was higher in the administration group than in the control group (FIG. 7B). It was observed, also from the photographs in FIG. 7C, that psoriasis-like symptoms (thickness and inflammation) of the ears in the administration group were suppressed as compared with those in the control group. These results demonstrated the efficacy of the nucleic acid constructs (mRNA vaccines) comprising the polynucleotide encoding the T cell epitopes designed in Example 1.

[Sequence Listing]

## Claims

1. A nucleic acid construct that induces production of an antibody against a target protein, the nucleic acid construct comprising: a polynucleotide encoding two or more T cell epitopes; and a polynucleotide encoding one or more B cell epitopes of the target protein.

2. The nucleic acid construct according to claim 1, wherein the two or more T cell epitopes each consist of an amino acid sequence selected from the group consisting of the amino acid sequences set forth in SEQ ID NOs: 1 to 82, or an amino acid sequence substantially identical with the amino acid sequence.

3. The nucleic acid construct according to claim 1 or 2, wherein the target protein is a protein produced in a subject's body.

4. The nucleic acid construct according to claim 1 or 2, wherein the target protein is IL-17A and/or IL-23.

5. The nucleic acid construct according to claim 1 or 2, wherein the target protein is a protein that causes a disease or a protein that is produced due to a disease, and wherein the disease is an autoimmune disease or an allergic disease.

6. The nucleic acid construct according to claim 5, wherein the autoimmune disease is one or more autoimmune diseases selected from the group consisting of psoriasis, rheumatoid arthritis, systemic lupus erythematosus, Graves' disease, chronic thyroiditis, type I diabetes, vasculitis, Addison's disease, polymyositis, Sjogren's syndrome, systemic sclerosis and glomerulonephritis.

7. The nucleic acid construct according to claim 5, wherein the allergic disease is one or more allergic diseases selected from the group consisting of atopic dermatitis, bronchial asthma, allergic rhinitis, hay fever, food allergy and allergic conjunctivitis.

8. The nucleic acid construct according to claim 1 or 2, which is DNA or RNA.

9. An agent for preventing or treating a disease, comprising the nucleic acid construct according to claim 1 or 2 as an active ingredient.

10. The agent according to claim 9, wherein the disease is an autoimmune disease or an allergic disease.

11. The agent for preventing a disease according to claim 9, which is a pan-HLA compatible vaccine.

12. A method for producing an agent for preventing or treating a disease, the method comprising the step of using a nucleic acid construct that induces production of an antibody against a target protein as an active ingredient, wherein the nucleic acid construct comprises: a polynucleotide encoding two or more T cell epitopes; and a polynucleotide encoding one or more B cell epitopes of the target protein.

13. The production method according to claim 12, wherein the disease is an autoimmune disease or an allergic disease.

14. A method for designing a nucleic acid construct or protein complex that induces production of an antibody against a target protein, the method comprising the step of combining two or more T cell epitopes and one or more B cell epitopes of the target protein.

15. A method for producing an agent for preventing or treating a disease, the method comprising the following steps:
(P) the step of carrying out the design method according to claim 14 to design a nucleic acid construct or protein complex that induces production of an antibody against a target protein; and
(Q) the step of preparing the nucleic acid construct or protein complex designed in the step (P).
